# EUROPEAN PATENT SPECIFICATION

(11) **EP 4 101 864 B1**
(45) Date of publication and mention of the grant of the patent: **22.10.2025**
(21) Application number: 21749986.2
(22) Date of filing: 11.01.2021
(51) Int. Cl.: C07K 14/47, A61K 38/17, A61K 47/54, A61P 31/14, A61P 31/18, A61K 38/00, C07K 14/005

(54) **POLYPEPTIDE, AND PREPARATION METHOD THEREFOR AND USE THEREOF**
POLYPEPTID SOWIE HERSTELLUNGSVERFAHREN DAFÜR UND VERWENDUNG DAVON
POLYPEPTIDE, SON PROCÉDÉ DE PRÉPARATION ET SON UTILISATION

(30) Priority: 05.02.2020 CN 202010080751
(43) Date of publication of application: 14.12.2022
(73) Proprietor: Shanxi Jinbo Bio-Pharmaceutical Co., Ltd., Shanxi 030032 (CN)
(72) Inventor: LU, Lu, Shanghai 200433 (CN); JIANG, Shibo, Shanghai 200433 (CN); XIA, Shuai, Shanghai 200433 (CN); YANG, Xia, Shanghai 200433 (CN)
(74) Representative: Guardian IP Consulting I/S
(86) International application number: PCT/CN2021/071078
(87) International publication number: WO 2021/155733

(56) References cited:
- WO-A1-2018/176103
- WO-A2-2005/007671
- WO-A2-2005/063801
- WO-A2-2005/077103
- CN-A- 106 749 558
- CN-A- 107 022 008
- XIA SHUAI; ZHU YUN; LIU MEIQIN; LAN QIAOSHUAI; XU WEI; WU YANLING; YING TIANLEI; LIU SHUWEN; SHI ZHENGLI; JIANG SHIBO; LU LU: "Fusion mechanism of 2019-nCoV and fusion inhibitors targeting HR1 domain in spike protein", CELLULAR & MOLECULAR IMMUNOLOGY, CHINESE SOCIETY OF IMMUNOLOGY,, CH, vol. 17, no. 7, 11 February 2020 (2020-02-11), CH, pages 765 - 767, XP037183146, ISSN: 1672-7681, DOI: 10.1038/s41423-020-0374-2
- XIA SHUAI, YAN LEI, XU WEI, AGRAWAL ANURODH SHANKAR, ALGAISSI ABDULLAH, TSENG CHIEN-TE K., WANG QIAN, DU LANYING, TAN WENJIE, WILS: "A pan-coronavirus fusion inhibitor targeting the HR1 domain of human coronavirus spike", SCIENCE ADVANCES, AMERICAN ASSOCIATION FOR THE ADVANCEMENT OF SCIENCE, US, vol. 5, no. 4, 10 April 2019 (2019-04-10), US, pages eaav4580, XP055783044, ISSN: 2375-2548, DOI: 10.1126/sciadv.aav4580
- XIA SHUAI, YAN LEI, XU WEI, AGRAWAL ANURODH SHANKAR, ALGAISSI ABDULLAH, TSENG CHIEN-TE K., WANG QIAN, DU LANYING, TAN WENJIE, WILS: "A pan-coronavirus fusion inhibitor targeting the HR1 domain of human coronavirus spike", SCIENCE ADVANCES, AMERICAN ASSOCIATION FOR THE ADVANCEMENT OF SCIENCE, US, vol. 5, no. 4, 10 April 2019 (2019-04-10), US, pages eaav4580, XP055783044, ISSN: 2375-2548, DOI: 10.1126/sciadv.aav4580
- LIU, S. XIAO, G. CHEN, Y. HE, Y. NIU, J. ESCALANTE, C.R. XIONG, H. FARMAR, J. DEBNATH, A.K. TIEN, P. JIANG, S.: "Interaction between heptad repeat 1 and 2 regions in spike protein of SARS-associated coronavirus: implications for virus fusogenic mechanism and identification of fusion inhibitors", THE LANCET, ELSEVIER, AMSTERDAM, NL, vol. 363, no. 9413, 20 March 2004 (2004-03-20), AMSTERDAM, NL, pages 938 - 947, XP005066763, ISSN: 0140-6736, DOI: 10.1016/S0140-6736(04)15788-7
- LU LU, LIU QI, ZHU YUN, CHAN KWOK-HUNG, QIN LILI, LI YUAN, WANG QIAN, CHAN JASPER FUK-WOO, DU LANYING, YU FEI, MA CUIQING, YE SHEN: "Structure-based discovery of Middle East respiratory syndrome coronavirus fusion inhibitor", NATURE COMMUNICATIONS, vol. 5, no. 1, 1 May 2014 (2014-05-01), XP055799923, DOI: 10.1038/ncomms4067

## Description

This application claims the priority of the Chinese invention patent application filed on February 5, 2020, titled "Polypeptide, preparation method and use thereof" with the application number of 202010080751.4.

### Field of the Invention

The present invention belongs to the field of polypeptide medicine, and specifically relates to a polypeptide that inhibits coronavirus.

### Background of the Invention

The novel coronavirus may be transmitted to humans by certain animals. The pathogen was quickly identified as a new type of coronavirus, and the World Health Organization named it as 2019-nCoV [J. Med. Virol. 2020. 92, doi: 10.1002/jmv.25678]. Subsequently, the International Committee on Taxonomy of Viruses named the novel coronavirus as SARS-CoV-2.

Therefore, how to effectively prevent and control the spread of the virus and developing specific drugs have become important tasks that need to be solved urgently.

WO2005/077103 discloses compositions and methods for modification and prevention of SARS coronavirus infectivity, including SARS-CoV-derived peptides and PEGylated derivates thereof.

CN107022008A discloses SARS-CoV-derived peptides capable of inhibiting human coronavirus infections, and applications thereof.

### Summary of the Invention

The present invention discloses a polypeptide that can inhibit the infection of a novel coronavirus (2019-nCoV). The inventors found a polypeptide or polypeptide derivative that can inhibit 2019-nCoV infection alone based on the HR1 target in the S2 region of the S protein on 2019-nCoV. They can also have a good inhibitory effect on other coronaviruses that may infect humans, such as SARS-like viruses (Rs3367-CoV or WIV1-CoV). The present invention can provide a good preventive and therapeutic candidate drug for 2019-nCoV that is still circulating at present and SARS-like viruses that may break out in the future.

In one aspect, the present invention provides a polypeptide set forth as SEQ ID NO. 2 for use in inhibiting 2019-nCoV or treating and/or preventing a disease caused by 2019-nCoV.

In one aspect, the present invention further provides a fusion protein or conjugate comprising the polypeptide set forth as SEQ ID NO. 2 for use in inhibiting 2019-nCoV or treating and/or preventing a disease caused by 2019-nCoV.

In one aspect, the present invention further provides a nucleic acid encoding the polypeptide set forth as SEQ ID NO. 2 or the fusion protein comprising the polypeptide set forth as SEQ ID NO. 2 for use in inhibiting 2019-nCoV or treating and/or preventing a disease caused by 2019-nCoV.

In one aspect, the present invention further provides a vector comprising the nucleic acid as defined above for use in inhibiting 2019-nCoV or treating and/or preventing a disease caused by 2019-nCoV.

In one aspect, the present invention further provides a host cell comprising the vector as defined above for use in inhibiting 2019-nCoV or treating and/or preventing a disease caused by 2019-nCoV.

A method for preparing the polypeptide or the fusion protein as defined above is disclosed, which comprises the following steps:
(1) introducing the vector as defined above into a host cell;
(2) culturing the host cell in a suitable medium; and
(3) harvesting and isolating the polypeptide or the fusion protein.

In one aspect, the present invention further provides a polypeptide derivative of SEQ ID NO. 2, which is a derivative obtained by palmitoylation modification or cholesterol modification of the polypeptide,
wherein the palmitoylation modification or cholesterol modification is performed at the C-terminus of the polypeptide, wherein the polypeptide is linked to palmitoylation modified lysine or cholesterol modified cysteine via a linker at the C-terminus, and wherein the linker is PEGylated.

In one embodiment, the polypeptide is linked to the palmitic acid or cholesterol moiety via a linker at the C-terminus.

For example, the linker can be PEGylated with dPEG.

In other words, the polypeptide is linked to palmitic acid or cholesterol moiety via a linker via lysine or cysteine.

In one embodiment, the linker comprises (GSG)n or (GSGSG)n, wherein n may be any integer. For example, n is 1, 2, 3, 4, or 5.

In one embodiment, the linker is -(GSG)n-DPEG4- or -(GSGSG)n-DPEG4-, wherein n may be any integer. For example, n is 1, 2, 3, 4, or 5.

In one embodiment, the polypeptide derivative is SLDQINVTFLDLEYEMKKLEEAIKKLEESYIDLKEL-(GSG)n-DPEG4-K-palmitic acid or
SLDQINVTFLDLEYEMKKLEEAIKKLEESYIDLKEL-(GSGSG)n-DPEG4-C-cholesterol. N may be any integer. For example, n is 1, 2, 3, 4, or 5.

In one embodiment, the polypeptide derivative is SLDQINVTFLDLEYEMKKLEEAIKKLEESYIDLKEL-GSG-DPEG4-K-palmitic acid or
SLDQINVTFLDLEYEMKKLEEAIKKLEESYIDLKEL-GSGSG-DPEG4-C-cholesterol.

In one aspect, the present invention further provides a composition comprising the polypeptide as defined above for use in inhibiting 2019-nCoV or treating and/or preventing a disease caused by 2019-nCoV or a composition comprising the derivative of the present invention.

In one embodiment, the composition is an external preparation. In one embodiment, the composition is an external liniment. In one embodiment, the composition is an external gel or external infiltration preparation.

In one embodiment, the composition further comprises an agent for inhibiting coronavirus or treating and/or preventing a disease caused by coronavirus. The agent can be selected from the group consisting of favipiravir, nelfinavir, arbidol, lopinavir, ritonavir, darunavir, chloroquine phosphate, or remdesivir. In one embodiment, the composition comprises a pharmaceutically acceptable carrier.

Also disclosed is the use of the polypeptide, the fusion protein or conjugate comprising the polypeptide, the nucleic acid encoding the polypeptide or the fusion protein, the vector comprising the nucleic acid, the host cell comprising the nucleic acid, or the composition comprising the polypeptide as defined above in the manufacture of a medicament for inhibiting 2019-nCoV or treating and/or preventing a disease caused by 2019-nCoV.

Further disclosed is the use of the polypeptide derivative or the composition comprising the polypeptide derivative of the present invention in the manufacture of a medicament for inhibiting coronavirus or treating and/or preventing a disease caused by coronavirus.

In one embodiment, the coronavirus is 2019-nCoV, Rs3367-CoV and/or WIV1-CoV.

In one embodiment, the dosage form of the medicament is tablet, capsule, dripping pill, aerosol, pill, powder, solution, suspension, emulsion, granule, liposome, transdermal agent, suppository or lyophilized powder injection.

In one embodiment, the medicament is preferably administered by the followings: injection, including subcutaneous injection, intravenous injection, intramuscular injection and intraperitoneal injection, intracisternal injection or infusion, etc., cavity administration, such as rectal, vaginal and sublingual, respiratory administration, such as through the nasal cavity; mucosal administration, or topical administration.

In one aspect, the present invention provides the polypeptide, the fusion protein or conjugate comprising the polypeptide, the nucleic acid encoding the polypeptide or the fusion protein, the vector comprising the nucleic acid, the host cell comprising the nucleic acid, or the composition comprising the polypeptide as defined above for use in inhibiting 2019-nCoV in vitro.

In one aspect, the present invention provides the polypeptide derivative or the composition comprising the polypeptide derivative of the present invention for use in inhibiting coronavirus in vitro.

In one embodiment, the coronavirus is 2019-nCoV, Rs3367-CoV or WIV1-CoV.

### Brief Description of the Drawings

Figure 1: A diagram of sequence analysis of 2019-nCoV and SARS-like virus S protein.
Figure 2: Inhibitory activity of the polypeptide on cell-cell fusion mediated by the S protein of 2019-nCoV.
Figure 3: Inhibitory activity of the polypeptide on the 2019-nCoV pseudovirus.
Figure 4: Inhibitory activity of the polypeptide on the SARS-CoV pseudovirus.
Figure 5: Inhibitory activity of the polypeptide on the Rs3367-CoV pseudovirus.
Figure 6: Inhibitory activity of the polypeptide on the WIV1-CoV pseudovirus.
Figure 7: Inhibitory activity of the polypeptide on the live 2019-nCoV virus.
Figures 8a-8b: 2019-HR2P represents the antiviral mechanism of the polypeptide.
Figure 9: pAAV-IRES-EGFP plasmid map.
Figure 10: Inhibitory effect of polypeptides EK1 and EK1-chol on infection of the SARS-CoV-2 live virus.
Figure 11: Preventive and therapeutic protective effects of EK1 polypeptide in vivo. A. changes in lung viral load; B. changes in enteric viral load.
Figure 12: Preventive and therapeutic protective effects of EK1-chol polypeptide in vivo. A. changes in lung viral load; B. changes in enteric viral load.

### Detailed Description of the Invention

For the current outbreak of 2019-nCoV, there are currently no specific drugs targeting the virus, including polypeptide inhibitors for the S2 region. The present invention first designed a set of polypeptide inhibitors targeting this region based on the sequence characteristics of the HR1 target site on the S2 region of the virus and the feature that key amino acid sites are relatively conservative. The experimental results found that these polypeptides can effectively inhibit the virus membrane fusion and pseudovirus infection, and have the potential to be developed into highly effective anti-2019-nCoV specific drugs. At the same time, we also found that the designed polypeptide can also effectively inhibit a variety of SARS-like viruses from bats. Therefore, the present invention can provide a good preventive and therapeutic candidate drug for the 2019-nCoV that is still circulating and the SARS-like viruses that may break out in the future.

The purpose of the present invention is to provide a set of entry inhibitory polypeptides that can inhibit 2019-nCoV and SARS-like virus infections. The entry inhibitory polypeptides bind to the HR1 region in the S2 protein of 2019-nCoV and SARS-like viruses, and interferes with the 6HB formation process of the virus, thereby inhibiting the fusion and infection processes of the virus. The sequences of this set of polypeptides are shown in Table 4, or detailed in the polypeptide sequence listing. The important innovation point of this invention is that the polypeptides have inhibitory activity against newly outbroken 2019-nCoV, and the inhibitory activity of some polypeptides has reached an unexpectedly high level.

One of the objectives of the present invention is to provide a polypeptide for use in inhibiting 2019-nCoV or treating and/or preventing a disease caused by 2019-nCoV; or a polypeptide derivative that inhibits coronavirus or has an inhibitory function on coronavirus infection. This is achieved by binding to the HR1 region in the S2 protein of the coronavirus. The coronavirus can be 2019-nCoV, SARS-like virus Rs3367-CoV or WIV1-CoV.

Herein, the polypeptide may be a polypeptide set forth as SEQ ID NO: 2.

Amino acid addition refers to the addition of amino acids to the C-terminus or N-terminus of an amino acid sequence, such as SEQ ID NO: 2, as long as the polypeptide has inhibitory activity against coronavirus.

Amino acid substitution refers to the replacement of a certain amino acid residue at a certain position in the amino acid sequence, such as SEQ ID NO: 2, by another amino acid residue, as long as the polypeptide has inhibitory activity against coronavirus.

Amino acid insertion refers to the insertion of amino acid residues at appropriate positions in the amino acid sequence, such as the sequence of SEQ ID NO: 2. The inserted amino acid residues may be either adjacent to each other in whole or in part, or not adjacent to each other, as long as the polypeptide has inhibitory activity against the coronavirus.

Amino acid deletion refers to the deletion of 1, 2, 3 or more amino acids from the amino acid sequence, such as the sequence of SEQ ID NO: 2, as long as the polypeptide has inhibitory activity against the coronavirus.

The substitution may be a conservative amino acid substitution, which means that compared with the amino acid sequence of SEQ ID NO: 2, 3, preferably 2 amino acids or 1 amino acid are replaced by amino acids with similar properties to form peptides. These conservative variant peptides can be produced by amino acid substitutions according to Table 1.

Conservative substitutions can be defined in terms of substitutions within the amino acid classes reflected in one or more of the following three tables:

**Table 1: Amino acid residue classes for conservative substitutions**

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

**Table 2: Alternative conservative amino acid residue substitution classes**

| | | | |
|---|---|---|---|
| 1 | A | S | T |
| 2 | D | E | |
| 3 | N | Q | |
| 4 | R | K | |
| 5 | I | L | M |
| 6 | F | Y | W |

**Table 3: Alternative physical and functional classifications of amino acid residues**

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | Q, T, K, S, G, N, D, E, and R |

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

Disclosed are a fusion protein or conjugate comprising the polypeptide set forth as SEQ ID NO.: 2, as long as the fusion protein or conjugate has the coronavirus inhibitory activity of the polypeptide. Those skilled in the art are fully aware of the preparation methods of fusion proteins or conjugates. For example, the polypeptide can be fused or conjugated to another functional moiety directly or indirectly through a linker. Another functional moiety can be a cholesterol moiety, palmitic acid moiety, carrier protein, active protein, marker protein and the like. The polypeptide can be modified by myristoylation, or stearic acid, or palmitic acid, or cholesterol, amidation, or isoprenylation.

The polypeptide may be produced synthetically or the polypeptide may be expressed by cells. For example, the polypeptide can be synthesized by chemical means. Alternatively, the polypeptide can be expressed in recombinant cells. The type of cell is not limited, for example, the cell may be a eukaryotic cell or a prokaryotic cell. Eukaryotic cells may be fungal cells, such as yeast cells, or insect cells or mammalian cells, such as mouse cells. The prokaryotic cell may be a bacterial cell, such as an E. coli cell.

The nucleic acid encoding the polypeptide can be codon optimized according to the host cell used. The nucleic acid can be cloned into a suitable expression vector, which can be then introduced into a host cell for expression. The type of expression vector is not limited and is well known to those skilled in the art.

Method for preparing the polypeptide or fusion protein comprises introducing the expression vector into the host cells, which are then cultured in a suitable medium, and then harvesting or isolating the polypeptides or fusion proteins in the supernatant, or in the context of intracellular expression, the host cells can be collected, and then the cells can be lysed to collect these polypeptides or fusion proteins. In the context of a polypeptide or fusion protein, the polypeptide or fusion protein can be linked to a suitable signal peptide. The signal peptide is cleaved or not after harvest. These techniques are well known to those skilled in the art.

The polypeptides can be prepared in the form of derivatives. For example, the polypeptide can be modified by palmitoylation or cholesterol modification. The method of palmitoylation modification or cholesterol modification is well known. In one embodiment, palmitoylation or cholesterol modification can be performed at the C-terminus of the polypeptide, so that the polypeptide is linked to palmitic acid or cholesterol moiety at the C-terminus. The linkage can be a direct connection or can be connected through a linker.

The type and length of the linker can vary. For example, the linker can be (GSG)n or (GSGSG)n, wherein n can be any integer, such as 1, 2, 3, 4, 5, 6, and so on.

The linker can be PEGylated. The means of PEGylation modification are known to those skilled in the art. As used herein, the term PEGylated linker refers to the linker with one or more PEG attached. Here, the PEGylated linker can be PEGylated (GSG)n or (GSGSG)n. n can be any integer, such as 1, 2, 3, 4, 5, 6, and so on.

As used herein, "DPEG" refers to discrete polyethylene glycol. DPEG4 indicates that the number of repeated ethylene glycol residues is 4.

As used herein, the coronavirus can be any kind of coronavirus. Preferably, the coronavirus is 2019-nCoV, Rs3367-CoV or WIV1-CoV.

The polypeptides or the polypeptide derivatives of the present invention can be used alone or in combination, or in combination with other agents having anti-coronavirus activity or coronavirus inhibitory activity. For example, these agents may be agents that have been reported to have inhibitory activity against coronaviruses or have therapeutic effects against coronavirus diseases, such as coronavirus pneumonia, such as favipiravir, nelfinavir, arbidol, lopinavir, ritonavir, chloroquine phosphate, darunavir or remdesivir, and so on.

Herein, the polypeptide for use in inhibiting 2019-nCoV or treating and/or preventing a disease caused by 2019-nCoV or the polypeptide derivative of the present invention may include 2019-HR2P, EK1, EK1-plam, and EK1-chol. They can be prepared in the form of a composition for application. The composition may comprise a suitable carrier, such as a pharmaceutically acceptable carrier. Such a composition can be used for external use, for example, as an external preparation, an external liniment, such as an external gel or external infiltration preparation. Such a composition can be coated on articles that need to inhibit viruses, such as, but not limited to, masks, tissues, gloves, clothing, such as protective clothing. Alternatively, it can be added as an active ingredient to hand washing supplies, such as hand sanitizer, shower gel and the like. Such polypeptides or polypeptide derivatives can be used to inhibit coronavirus in vitro to prevent and/or reduce viral infections. Such polypeptides or polypeptide derivatives can be used to prevent or treat coronavirus infections or diseases caused by coronaviruses in subjects.

The polypeptides for use in inhibiting 2019-nCoV or treating and/or preventing a disease caused by 2019-nCoV or the polypeptide derivatives of the present invention can also be formulated into medicines or pharmaceutical compositions. Such medicine can be tablet, capsule, dripping pill, aerosol, pill, powder, solution, suspension, emulsion, granule, liposome, transdermal agent, suppository or lyophilized powder injection. These medicines or pharmaceutical compositions can be applied by various administration methods, such as injection, including subcutaneous injection, intravenous injection, intramuscular injection and intraperitoneal injection, intracisternal injection or infusion, etc., cavity administration, such as rectal, vaginal and sublingual, respiratory administration, such as through the nasal cavity; mucosal administration, or topical administration, etc.

The present invention provides a polypeptide set forth as SEQ ID NO. 2, a fusion protein or conjugate comprising the polypeptide, a nucleic acid encoding the polypeptide or the fusion protein, a vector comprising the nucleic acid, a host cell comprising the nucleic acid or a composition comprising the polypeptide for use in inhibiting 2019-nCoV or treating and/or preventing a disease caused by 2019-nCoV in a subject.

The present invention also relates to a polypeptide derivative of SEQ ID NO. 2, which is a derivative obtained by palmitoylation modification or cholesterol modification of the polypeptide, wherein the polypeptide is linked to palmitoylation modified lysine or cholesterol modified cysteine via a linker at the C-terminus, wherein the linker is PEGylated, or a composition comprising the polypeptide derivative, for use in method for preventing or treating a coronavirus infection or a disease caused by a coronavirus in a subject. Preferably, the coronavirus is 2019-nCoV, Rs3367-CoV or WIV1-CoV.

### Examples

The present invention is further illustrated by the following examples, but any examples or combinations thereof should not be construed as limiting the scope or implementation of the present invention. The scope of the present invention is defined by the appended claims. In combination with this specification and common knowledge in the field, a person of ordinary skill in the art can clearly understand the scope defined by the claims.

### Experimental Materials:

### Polypeptides:

The inventors designed three artificial polypeptides derived from the HR2 region by comparing the homology of the HR1 and HR2 sequences in the human coronavirus spike protein. The polypeptides simply referred to as a series of 2019-HR2P polypeptides (2019-HR2P, EK1-Plam and EK1-chol). The specific sequences and structures of these polypeptides are shown in Table 1. They are synthesized and purified by Shanghai SynPeptide Co Ltd, with purity of >95%.

**Table 4. Specific sequences of the polypeptides used in the experiments:**

| **Name of polypeptide** | **Sequence of polypeptide** |
|---|---|
| **2019-HR2P:** | **DISGINASVVNIQKEIDRLNEVAKNLNESLIDLQEL (SEQ ID NO. 1)** |
| **EK1** | **SLDQINVTFLDLEYEMKKLEEAIKKLEESYIDLKEL (SEQ ID No. 2)** |
| **EK1-plam** | **SLDQINVTFLDLEYEMKKLEEAIKKLEESYIDLKEL-GSG-DPEG4-K(palmitic)** |
| **EK1-chol** | **SLDQINVTFLDLEYEMKKLEEAIKKLEESYIDLKEL-GSGSG-DPEG4-C(cholesterol)** |
| **2019-HR1P** | **ANQFNSAIGKIQDSLSSTASALGKLQDVVNQNAQALNTLVKQ (SEQ ID No. 3)** |

The amino acid sequence of 2019-nCoV-S in pAAV-IRES-EGFP-2019-nCoV-S (the restriction sites for insertion are BamHI and XhoI) is as follows:

MPMGSLQPLATLYLLGMLVASVLAQCVNLTTRTQLPPAYTNSFTRGVYYPDKVFRSS
VLHSTQDLFLPFFSNVTWFHAIHVSGTNGTKRFDNPVLPFNDGVYFASTEKSNIIRGWI
FGTTLDSKTQSLLIVNNATNVVIKVCEFQFCNDPFLGVYYHKNNKSWMESEFRVYSSA
NNCTFEYVSQPFLMDLEGKQGNFKNLREFVFKNIDGYFKIYSKHTPINLVRDLPQGFSA
LEPLVDLPIGINITRFQTLLALHRSYLTPGDSSSGWTAGAAAYYVGYLQPRTFLLKYNE
NGTITDAVDCALDPLSETKCTLKSFTVEKGIYQTSNFRVQPTESIVRFPNITNLCPFGEV
FNATRFASVYAWNRKRISNCVADYSVLYNSASFSTFKCYGVSPTKLNDLCFTNVYAD
SFVIRGDEVRQIAPGQTGKIADYNYKLPDDFTGCVIAWNSNNLDSKVGGNYNYLYRLF
RKSNLKPFERDISTEIYQAGSTPCNGVEGFNCYFPLQSYGFQPTNGVGYQPYRVVVLSF
ELLHAPATVCGPKKSTNLVKNKCVNFNFNGLTGTGVLTESNKKFLPFQQFGRDIADTT
DAVRDPQTLEILDITPCSFGGVSVITPGTNTSNQVAVLYQDVNCTEVPVAIHADQLTPT
WRVYSTGSNVFQTRAGCLIGAEHVNNSYECDIPIGAGICASYQTQTNSPRRARSVASQS
IIAYTMSLGAENSVAYSNNSIAIPTNFTISVTTEILPVSMTKTSVDCTMYICGDSTECSNL
LLQYGSFCTQLNRALTGIAVEQDKNTQEVFAQVKQIYKTPPIKDFGGFNFSQILPDPSK
PSKRSFIEDLLFNKVTLADAGFIKQYGDCLGDIAARDLICAQKFNGLTVLPPLLTDEMI
AQYTSALLAGTITSGWTFGAGAALQIPFAMQMAYRFNGIGVTQNVLYENQKLIANQF
NSAIGKIQDSLSSTASALGKLQDVVNQNAQALNTLVKQLSSNFGAISSVLNDILSRLDK
VEAEVQIDRLITGRLQSLQTYVTQQLIRAAEIRASANLAATKMSECVLGQSKRVDFCG
KGYHLMSFPQSAPHGVVFLHVTYVPAQEKNFTTAPAICHDGKAHFPREGVFVSNGTH
WFVTQRNFYEPQIITTDNTFVSGNCDVVIGIVNNTVYDPLQPELDSFKEELDKYFKNHT
SPDVDLGDISGINASVVNIQKEIDRLNEVAKNLNESLIDLQELGKYEQYIKWPWYIWLG
FIAGLIAIVMVTIMLCCMTSCCSCLKGCCSCGSCCKFDEDDSEPVLKGVKLHYTGGTET
SQVAPA (SEQ ID NO. 4)。

The nucleotide sequence is as follows:

GGATCCGCCACCATGCCCATGGGGTCTCTGCAACCGCTGGCCACCTTGTACCTGCT
GGGGATGCTGGTCGCTTCCGTGCTAGCCCAGTGCGTGAACCTGACCACAAGGACC
CAGCTGCCCCCTGCCTATACCAATTCCTTCACACGGGGCGTGTACTATCCCGACAA
GGTGTTTAGAAGCTCCGTGCTGCACTCTACACAGGATCTGTTTCTGCCTTTCTTTAG
CAACGTGACCTGGTTCCACGCCATCCACGTGAGCGGCACCAATGGCACAAAGCGG
TTCGACAATCCAGTGCTGCCCTTTAACGATGGCGTGTACTTCGCCTCTACCGAGAA
GAGCAACATCATCAGAGGCTGGATCTTTGGCACCACACTGGACTCCAAGACACAG
TCTCTGCTGATCGTGAACAATGCCACCAACGTCGTGATCAAGGTGTGCGAGTTCCA
GTTTTGTAATGATCCTTTCCTGGGCGTGTACTATCACAAGAACAATAAGAGCTGGA
TGGAGTCCGAGTTTCGCGTGTATTCTAGCGCCAACAATTGCACATTTGAGTACGTG
TCCCAGCCATTCCTGATGGACCTGGAGGGCAAGCAGGGCAATTTCAAGAACCTGA

GGGAGTTCGTGTTTAAGAATATCGATGGCTACTTCAAGATCTACTCTAAGCACACC
CCAATCAACCTGGTGCGCGACCTGCCACAGGGCTTCAGCGCCCTGGAGCCACTGG
TGGATCTGCCCATCGGCATCAACATCACCCGGTTTCAGACACTGCTGGCCCTGCAC
AGAAGCTACCTGACACCTGGCGACTCCTCTAGCGGATGGACCGCAGGAGCTGCCG
CCTACTATGTGGGCTATCTGCAGCCAAGGACCTTCCTGCTGAAGTACAACGAGAAT
GGCACCATCACAGACGCAGTGGATTGCGCACTGGACCCCCTGAGCGAGACCAAGT
GTACACTGAAGTCCTTTACCGTGGAGAAGGGCATCTATCAGACATCCAATTTCAGG
GTGCAGCCCACCGAGTCTATCGTGCGCTTTCCCAATATCACAAACCTGTGCCCTTT
TGGCGAGGTGTTCAACGCAACCAGGTTCGCAAGCGTGTACGCATGGAATAGGAAG
CGGATCAGCAACTGCGTGGCCGACTATAGCGTGCTGTACAACTCCGCCTCTTTCAG
CACCTTTAAGTGCTATGGCGTGTCCCCCACAAAGCTGAATGACCTGTGCTTTACCA
ACGTGTACGCCGATTCTTTCGTGATCAGGGGCGACGAGGTGCGCCAGATCGCACC
AGGACAGACAGGCAAGATCGCAGACTACAATTATAAGCTGCCTGACGATTTCACC
GGCTGCGTGATCGCCTGGAACAGCAACAATCTGGATTCCAAAGTGGGCGGCAACT
ACAATTATCTGTACCGGCTGTTTAGAAAGTCTAATCTGAAGCCATTCGAGAGGGAC
ATCTCTACAGAGATCTACCAGGCAGGCAGCACCCCATGCAATGGAGTGGAGGGCT
TTAACTGTTATTTCCCTCTGCAGAGCTACGGCTTCCAGCCAACAAACGGCGTGGGC
TATCAGCCCTACCGCGTGGTGGTGCTGAGCTTTGAGCTGCTGCACGCACCTGCAAC
AGTGTGCGGACCAAAGAAGTCCACCAATCTGGTGAAGAACAAGTGCGTGAACTTC
AACTTCAACGGACTGACCGGCACAGGCGTGCTGACCGAGTCCAACAAGAAGTTCC
TGCCCTTTCAGCAGTTCGGCAGGGACATCGCAGATACCACAGACGCCGTGCGCGA
CCCTCAGACCCTGGAGATCCTGGACATCACACCATGCTCTTTCGGCGGCGTGAGCG
TGATCACACCTGGCACCAATACAAGCAACCAGGTGGCCGTGCTGTATCAGGACGT
GAATTGTACCGAGGTGCCCGTGGCAATCCACGCAGATCAGCTGACCCCTACATGG
CGGGTGTACAGCACCGGCTCCAACGTGTTCCAGACAAGAGCCGGATGCCTGATCG
GAGCAGAGCACGTGAACAATTCCTATGAGTGCGACATCCCTATCGGCGCCGGCAT
CTGTGCCTCTTACCAGACCCAGACAAACTCTCCAAGGAGAGCCCGGAGCGTGGCA
TCCCAGTCTATCATCGCCTATACAATGAGCCTGGGCGCCGAGAACAGCGTGGCCTA
CTCTAACAATAGCATCGCCATCCCTACCAACTTCACAATCTCCGTGACCACAGAGA
TCCTGCCAGTGTCCATGACCAAGACATCTGTGGACTGCACAATGTATATCTGTGGC
GATTCTACCGAGTGCAGCAACCTGCTGCTGCAGTACGGCAGCTTTTGTACCCAGCT
GAATAGAGCCCTGACAGGCATCGCCGTGGAGCAGGACAAGAACACACAGGAGGT
GTTCGCCCAGGTGAAGCAGATCTACAAGACCCCACCCATCAAGGACTTTGGCGGC
TTCAATTTTTCCCAGATCCTGCCCGATCCTTCCAAGCCTTCTAAGCGGAGCTTTATC
GAGGACCTGCTGTTCAACAAGGTGACCCTGGCCGATGCCGGCTTCATCAAGCAGT
ATGGCGATTGCCTGGGCGACATCGCCGCCAGAGACCTGATCTGTGCCCAGAAGTTT
AATGGCCTGACCGTGCTGCCTCCACTGCTGACAGATGAGATGATCGCACAGTACA
CAAGCGCCCTGCTGGCAGGCACCATCACATCCGGATGGACCTTCGGCGCAGGAGC
CGCCCTGCAGATCCCCTTCGCTATGCAGATGGCCTATCGGTTCAACGGCATCGGCG
TGACCCAGAATGTGCTGTACGAGAACCAGAAGCTGATCGCCAATCAGTTTAACTC
CGCCATCGGCAAGATCCAGGACAGCCTGTCCTCTACAGCCTCCGCCCTGGGCAAG
CTGCAGGATGTGGTGAATCAGAACGCCCAGGCCCTGAATACCCTGGTGAAGCAGC
TGAGCTCCAACTTCGGCGCCATCTCTAGCGTGCTGAATGACATCCTGAGCCGGCTG
GACAAGGTGGAGGCAGAGGTGCAGATCGACCGGCTGATCACAGGCAGACTGCAG
TCTCTGCAGACCTACGTGACACAGCAGCTGATCAGGGCAGCAGAGATCAGGGCAA
GCGCCAATCTGGCAGCAACCAAGATGTCCGAGTGCGTGCTGGGCCAGTCTAAGAG
AGTGGACTTTTGTGGCAAGGGCTATCACCTGATGTCCTTCCCACAGTCTGCCCCTC
ACGGAGTGGTGTTTCTGCACGTGACCTACGTGCCAGCCCAGGAGAAGAACTTCAC
CACAGCACCAGCAATCTGCCACGATGGCAAGGCACACTTTCCCAGGGAGGGCGTG
TTCGTGAGCAACGGCACCCACTGGTTTGTGACACAGCGCAATTTCTACGAGCCTCA
GATCATCACCACAGACAATACATTCGTGTCCGGCAACTGTGACGTGGTCATCGGCA
TCGTGAACAATACCGTGTATGATCCTCTGCAGCCAGAGCTGGACAGCTTTAAGGA
GGAGCTGGATAAGTACTTCAAGAATCACACCTCCCCAGACGTGGATCTGGGCGAC

ATCAGCGGCATCAATGCCTCCGTGGTGAACATCCAGAAGGAGATCGACAGGCTGA
ACGAGGTGGCCAAGAATCTGAACGAGAGCCTGATCGATCTGCAGGAGCTGGGCAA
GTATGAGCAGTACATCAAGTGGCCCTGGTATATCTGGCTGGGCTTCATCGCCGGCC
TGATCGCTATCGTGATGGTGACCATCATGCTGTGCTGTATGACATCCTGCTGTTCTT
GCCTGAAGGGCTGCTGTAGCTGTGGCTCCTGCTGTAAGTTTGATGAGGACGATTCC
GAGCCAGTGCTGAAGGGCGTGAAGCTGCACTACACCGGCGGCACCGAGACATCTC
AGGTGGCCCCCGCCTAACTCGAG (SEQ ID NO. 5)

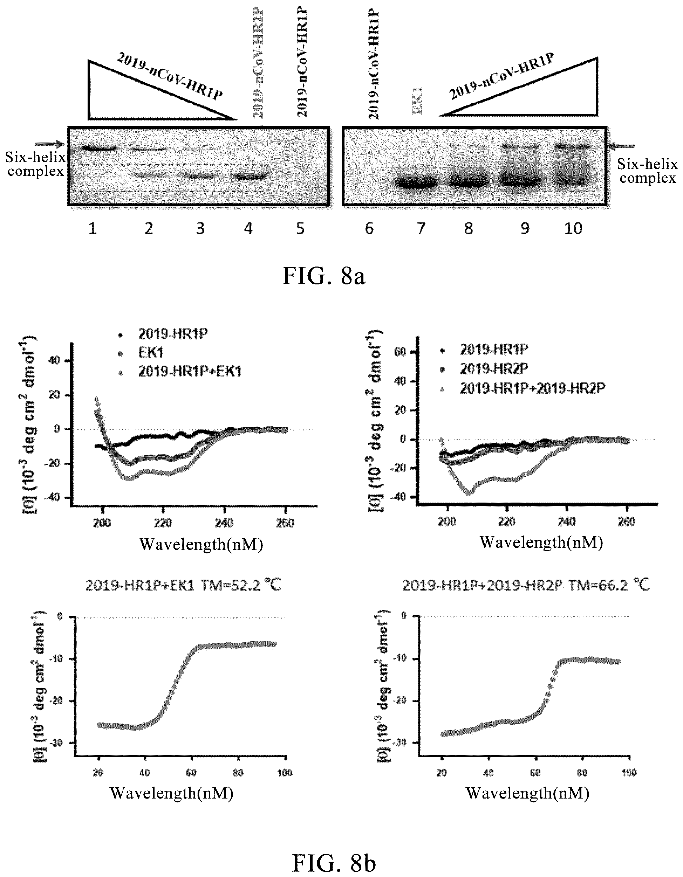

The plasmid map of pAAV-IRES-EGFP is shown in Figure 8. pAAV-IRES-EGFP was purchased from Agilent technologies in the United States.

pcDNA3.1-SARS-CoV-S plasmid, pcDNA3.1-Rs3367-CoV-S plasmid, pcDNA3.1-WIV1-CoV-S plasmid and pNL4-3.luc.RE plasmid were kindly donated by Dr. Du Lanying from the New York Blood Center, USA. The restriction sites for inserting 2019-nCoV-S into pcDNA3.1 are BamHI and XhoI.

The target cell HuH7 cell expressing the receptor of human coronavirus infection: purchased from the cell bank of the Chinese Academy of Sciences

### Example 1: Preparation of 2019-nCoV or SARS-CoV or Rs3367-CoV or WIV1-CoV pseudovirus.

### Experimental Method:

1. 293T cells were digested 24 hours before transfection and were plated in a 10 cm tissue culture dish (2x10⁶ /dish).
2. The culture medium was replaced with fresh pre-warmed DMEM medium (containing 10% FBS) 2 hours before transfection.
3. Two 1.5 mL EP tubes were used for transfection. 500 µL 0.9% NaCl solution was added to EP tube 1, which contained 20 µg of pcDNA3.1-plasmid (or pcDNA3.1-SARS-CoV-S plasmid or pcDNA3.1-Rs3367-CoV-S plasmid or pcDNA3.1-WIV1-CoV-S plasmid) and pNL4-3.luc.RE plasmid each (these plasmids were kindly provided by Dr. Du Lanying from the New York Blood Center, USA. The relevant information can also be found in the literature, Xia, S.#, Yan, L.#, Xu, W.#, Agrawal, A.S., Alsaissi, A., Tseng, C.T.K., Wang, Q., Du, L., Tan, W., Wilson, I.A.*, Jiang, S.*, Yang, B.*, Lu, L.* (2019). A pan-coronavirus Fusion Inhibitor Targeting the HR1 Domain of Human Coronavirus Spike. Science Advances. 2019 Apr 10;5(4):eaav4580). 500 µL 0.9% NaCl was also added to EP tube 2, and then 10 µL of transfection reagent vigofect was added. Both of the tubes were stood for 5 minutes.
4. 500 µL of the solution in EP tube 2 was added to EP tube 1 dropwise, mixed well with a pipette tip while being added, and left at room temperature for 15 minutes.
5. 1mL of mixed liquid obtained above was added dropwise and evenly to the 293T cell previously plated in culture dish.
6. After 8-10 hours of culture, the culture medium was replaced with 10 mL of fresh DMEM culture medium containing 10% FBS.
7. The supernatant containing pseudovirus was collected into the tubes after 48 hours;
8. The tubes were centrifuged at 4000 rpm for 4 mins to remove cell debris, filtered with a 0.45µm sterile filter, aliquoted and stored at -80°C for later use.

### Example 2: Inhibition of human coronavirus S protein-mediated cell-cell fusion

### Experimental Method:

1. 293T cells were transfected with the plasmid encoding the human coronavirus S protein and are cultured for 36-48 hours to be served as effector cells. Specifically, 293T cells were transfected with the plasmid pAAV-IRES-GFP-2019-nCoV-S encoding the 2019-nCoV S protein to obtain transfected cells, which are referred as 293T/2019/EGFP cells. 293T cells were transfected with the empty vector plasmid pAAV-IRES-GFP to obtain 293T/EGFP cells, which were used as negative control cells.
2. 293T/2019/EGFP cells and 293T/EGFP cells were digested with 0.02% EDTA, centrifuged, and resuspended in fresh DMEM medium containing 10% FBS to adjust cell concentration to 2x10⁵ cells/mL. 50 µL of 293T/2019/EGFP cells or 293T/EGFP cells was added into various test polypeptide drugs (50 µL) in serial dilutions, and incubated at 37°C for 30 min. Those without drugs were used as positive control cells.
3. 100 µL of the cell/drug mixture was added to the target cells (Huh-7) that had been plated on a 96-well plate, which was incubated in 5% CO2 at 37°C for 2-4 hours. The fusion status of the target cells was observed with the fluorescence microscope and recorded.

Calculation formula for inhibitory activity is: Inhibition rate = (number of fused cells in positive wells - number of fusion in drug wells) / (number of fused cells in positive wells - number of fusion in negative wells) * 100.

### Results and Discussion:

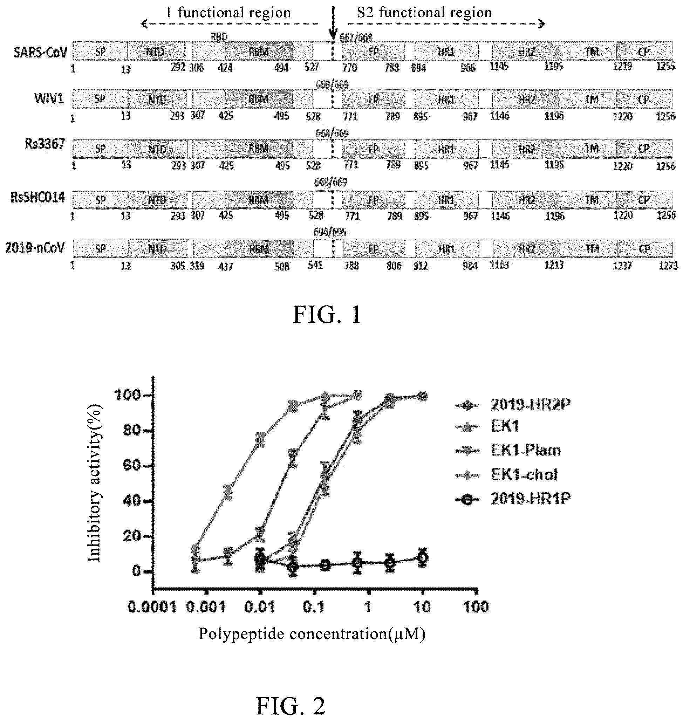

As shown in Figure 2, the series of 2019-HR2P polypeptides, 2019-HR2P, EK1-Plam, EK1-chol and EK1 have a very good inhibitory effect on the cell-cell fusion mediated by the S protein of 2019-nCoV. The results preliminarily show that the series of 2019-HR2P polypeptides can inhibit 2019-nCoV. For the cell-cell fusion mediated by the S protein of 2019-nCoV, compared with EK1, the inhibitory activity of its derivatives EK1-Plam and EK1-chol is significantly enhanced, and the polypeptide 2019-HR2P has considerable inhibitory activity.

### Example 3: Determination of the inhibitory activity of polypeptides against 2019-nCoV and other coronavirus pseudoviruses

### Experimental Method:

1. The test polypeptide was dissolved using DMSO and the polypeptide concentration was determined.
2. Cell suspension of target cells (HuH7 expressing the human infectious coronavirus receptor) was prepared, and adjusted the cell concentration in each well to 10⁴ cells.
3. The polypeptide drug was diluted in a 2-fold gradient using DMEM culture medium containing 10% FBS, with 50 µL per well.
4. A certain titer of 2019-nCoV or SARS-CoV or Rs3367-CoV or WIV1-CoV pseudovirus was added to the plate with the drug dilutions at 50 µL/well, which was then incubated at room temperature for 30 mins to allow the drug and virus fully interact. 100 µL of the drug and virus mixture was added to the target cells in each well from which the supernatant has been removed. After incubation for 12 hours at 37°C, they were replaced with fresh DMEM medium containing 10% FBS.
5. Luciferase activity was measured after 72 hours, the inhibition rate curve was produced. The half inhibitory concentration (IC50) of the drug was calculated.

Calculation formula for inhibitory activity: Inhibition rate = (value of virus control well - value of drug well) / (value of virus control well - value of cell well) * 100%. The virus control well is the well without drug, and the cell well is the well without virus.

### Results and Discussion:

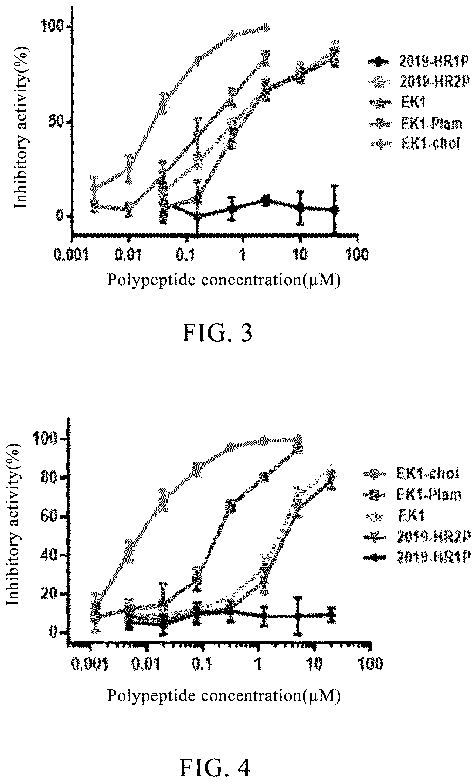

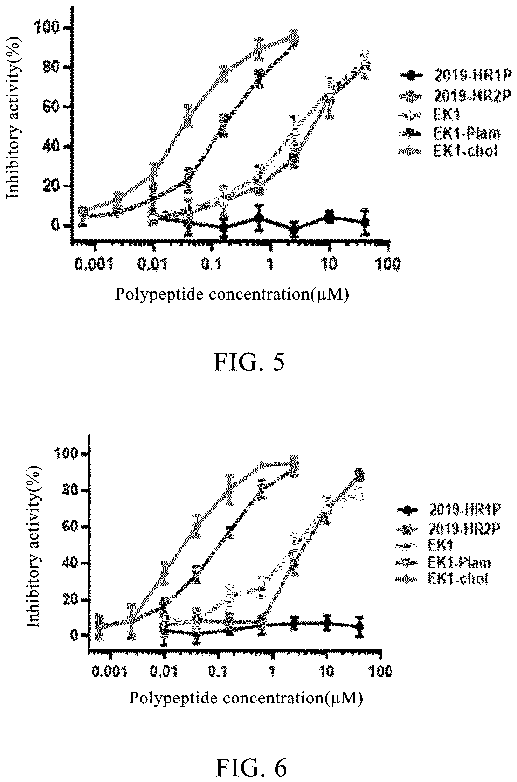

The antiviral activity of the series of 2019-HR2P polypeptides was tested on 2019-nCoV or SARS-CoV or Rs3367-CoV or WIV1-CoV pseudovirus system. The results are shown in Figures 3-6 and Table 5. The 2019-HR2P, EK1-Plam, EK1-chol and EK1 polypeptides also exhibited potent antiviral activity, which was consistent with the results of previous cell-cell fusion experiments. In addition, the inhibitory activity of EK1-Plam and EK1-chol polypeptides was significantly better than that of EK1 polypeptides, up to about tens of times.

**Table 5. The inhibitory activity of the series of 2019-HR2P polypeptides on a variety of human coronavirus pseudoviruses:**

| **Name of polypeptide** | **Inhibitory Activity, IC50(µM)** | | | |
|---|---|---|---|---|
| | **2019n-CoV** | **SARS-CoV** | **Rs3367** | **WIV1-CoV** |
| **2019-nCoV-HR2P** | **1.05** | **3.75** | **5.05** | **4.24** |
| **EK1** | **2.49** | **1.89** | **4.00** | **2.94** |
| **EK1-plam** | **0.36** | **0.17** | **0.13** | **0.10** |
| **EK1-chol** | **0.02** | **0.01** | **0.03** | **0.04** |

### Example 4: Determination of the inhibitory activity of polypeptides against live 2019-nCoV virus infection

### Experimental Method:

This study was authorized to be performed by the biosafety level 3 laboratory of Wuhan Institute of Virology, Chinese Academy of Sciences. In particular, the inhibitory activity of 2019-HR2P, EK1-Plam, EK1-chol and EK1 against the 2019-nCoV live virus was detected by the plaque reduction assay. The different concentrations of polypeptides as described above were first incubated with the live 2019-nCoV virus (virus titer of 100 PFU) for 30 minutes, and then they were added to the monolayer of VERO-E6 cells. After culturing at 37°C for 1 hour, the culture supernatant was removed. Then 0.9% methylcellulose was added to cover the cells. After 72 hours, the cells were stained to observe and count the number of plaques. Calculation formula for Inhibition activity: inhibition rate = (number of plaques in virus control wells - number of plaques in drug wells) / number of plaques in virus control wells * 100%.

### Results and Discussion:

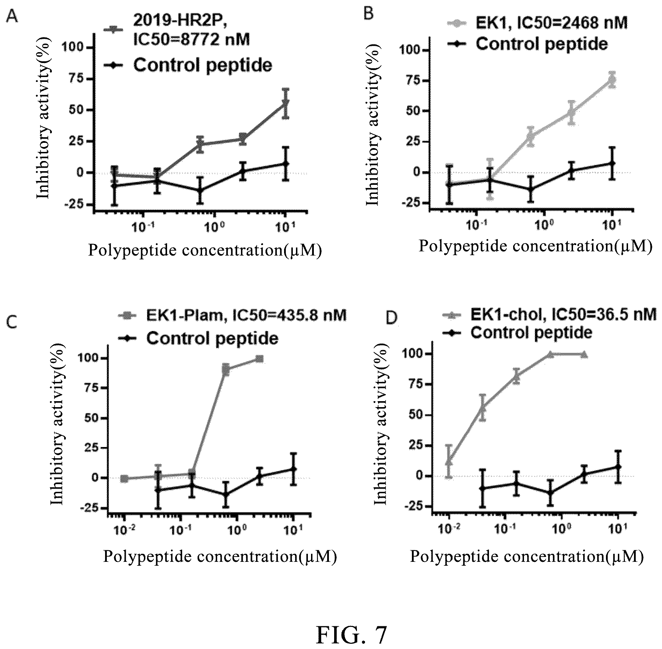

The antiviral activity of the series of 2019-HR2P polypeptides was detected on the 2019-nCoV live virus system. The results are shown in Figure 7. The 2019-HR2P, EK1-Plam, EK1-chol and EK1 polypeptides all exhibited effective the 2019-nCoV live virus inhibitory activities, while the control peptide did not exhibit inhibitory activity. In addition, the inhibitory activity of EK1-Plam and EK1-chol polypeptides were significantly better than that of EK1 polypeptide, which was 5 times and 67 times higher, respectively. Specifically, on the 2019-nCoV live virus system, the IC50 of EK1 is 2468 nM, the IC50 of EK1-Plam is 435.8 nM, and the IC50 of EK1-chol is 36.5 nM.

### Example 5: Study on the antiviral mechanism of the HR2 polypeptide

Taking the 2019-HR2P polypeptide as an example and EK1 polypeptide as a control, the interaction between the polypeptides and the polypeptide 2019-HR1P, which is derived from the HR1 region of 2019-nCoV, is detected, so as to further explore the antiviral mechanism of the 2019-HR2P polypeptide.

### Experimental Method:

1. Non-denaturing (Native) polyacrylamide gel electrophoresis (N-PAGE). The polypeptide 2019-HR1P belonging to the series of HR1 polypeptides was mixed with the polypeptide 2019-HR2P or EK1 belonging to the series of HR2 polypeptides (final concentration of 30 µM each), respectively, and was incubated at 37°C for 30 minutes. 5x loading buffer at a high pH was mixed with 4 times the volume of the peptide mixture, which was then loaded (25 µl per well) in 18% non-denaturing gel (Tiandz, Beijing) to conduct electrophoresis at 125V constant voltage for 2 hours at room temperature, and was stained with Coomassie brilliant blue.
2. Determination of the secondary structure of the polypeptide by circular dichroism spectroscopy. The alone polypeptide or polypeptide mixture was diluted with 50 mmol/L phosphate buffer, pH 7.2 to a final concentration of 10 µmol/L. The circular dichroism spectra were measured using a spectropolarimeter (Model J-815, Jasco Inc, Japan). The detection temperature was 4°C, with 5.0 nm of bandwidth, 0.1 nm of resolution, 0.1 cm of optical path, 4.0 s of reaction time, and 50 nm/min of scanning speed. The thermal denaturation of the polypeptide was monitored at 222 nm with a temperature gradient of 5°C/min. The blank control of the buffer solution was subtracted to correct the spectrum value. The melting curve was smoothed, and the midpoint temperature of the thermal dissociation transition, i.e., the Tm value, was calculated with Jasco.

### Results and Analysis:

1. After the 2019-HR2P is mixed with the 2019-HR1P polypeptide, a new band that may be a six-helix bundle complex is produced (Figure 8a, legend: lanes 1, 2, and 3 are 2019-nCoV-HR1P+2019-nCoV-HR2P; lane 4 is 2019-nCoV-HR2P; lane 5 is 2019-nCoV-HR1P). EK1 also showed a similar effect, proving the stability of the system and the formation of a six-helix bundle complex by EK1 (Figure 8a, legend: lane 6 is 2019-nCoV-HR1P; lane 7 is EK1; lanes 8, 9, 10 are EK1+ 2019-nCoV-HR1P).
2. A single polypeptide has a low helical structure. When the 2019-HR2P polypeptide is mixed with the 2019-HR1P polypeptide, a high helical structure appears. This result is consistent with the CD spectrum of the six-helix bundle (6-HB) structure. And its Tm value indicates its thermal stability. EK1 also showed a similar effect, proving the stability of the system and EK1 also formed a 6-HB complex.

### Discussion

In summary, the present invention found for the first time that the series of 2019-HR2P polypeptides (2019-HR2P, EK1, EK1-Plam and EK1-chol) have a good inhibitory activity against 2019-nCoV, and the mechanism of action is to form a stable heterologous 6HB by binding to the HR1 region of the virus, preventing the interaction of the viral HR2 and HR1 trimers, preventing the formation of homologous 6HB, thereby interfering with the fusion between the virus and the cell membrane and blocking the virus infection. In addition, it was also found that the series of 2019-HR2P polypeptides (2019-HR2P, EK, EK1-Plam and EK1-chol) have a good inhibitory activity against SARS viruses and SARS-like viruses (Rs3367-CoV and WIV1-CoV). It was found for the first time that the activities of EK1-Plam and EK1-chol are significantly higher than that of EK1, up to tens of times.

In the present invention, the inventors detected the inhibitory activity of the series of 2019-HR2P polypeptides (2019-HR2P, EK1-Plam and EK1-chol) on the corresponding virus fusion process by establishing a cell-cell fusion system mediated by 2019-nCoV and SARS-like virus S protein, as shown in Figure 2. In 2014, lu 1. (Lulu, one of the inventors of this patent) and others constructed a cell-cell fusion system for SARS-CoV, MERS-CoV, etc. by co-expressing the S protein of the virus and GFP, and successfully designed and evaluated a series of polypeptide entry inhibitors with better activity based on this well-recognized system for simulating virus infection. The results were published in Nature Communications and Science Advances. In the present invention, using the same method, the inventors took the lead in establishing a 2019-nCoV S protein-mediated membrane fusion system in the world, and separately detected the membrane fusion inhibitory effects of the series of polypeptides invented on 2019-nCoV and SARS-like viruses, with the polypeptide entry inhibitor EK1 having the best activity at present as a control (References: Lu, L., Q. Liu, Y. Zhu, K. H. Chan, L. Qin, Y. Li, Q. Wang, J. F. Chan, L. Du, F. Yu, C. Ma, S. Ye, K. Y. Yuen, R. Zhang, and S. Jiang. 2014. Structure-based discovery of Middle East respiratory syndrome coronavirus fusion inhibitor. Nat Commun 5:3067. and Xia, S., Yan, L., Xu, W., Agrawal, A.S., Alsaissi, A., Tseng, C.T.K., Wang, Q., Du, L., Tan, W., Wilson, I.A., Jiang, S., Yang, B., Lu, L. A pan-coronavirus Fusion Inhibitor Targeting the HR1 Domain of Human Coronavirus Spike. Science Advances. 2019 Apr 10;5(4):eaav4580). In the cell-cell fusion experiment of 2019-nCoV, all the series of 2019-HR2P polypeptides and EK1 polypeptides showed good inhibitory effects, and the inhibitory activity of EK1-Plam and EK1-chol polypeptides was significantly better than that of EK1 polypeptides, up to about 10-100 times (Figure 2). The negative control 2019-HR1P polypeptide showed no activity.

At the same time, in order to further determine the inhibitory activity of the polypeptide or polypeptide derivative of the present invention, the inventors also used another well-recognized model for evaluation of antiviral drug activity - pseudovirus infection model in the present invention. The inventors took the lead in establishing the 2019-nCoV pseudovirus infection system in the world, and then detected the inhibitory effect of the series of 2019-HR2P polypeptides and the control EK1 polypeptide on the pseudovirus. As shown in Figure 3, the series of 2019-HR2P polypeptides showed good inhibitory effects on both 2019-nCoV pseudoviruses and live viruses, and the inhibitory activities of EK1-Plam and EK1-chol polypeptides were significantly better than that of EK1 polypeptides, up to approximately 6-120 times (Figure 3 and Table 2). This result is consistent with the result of the MERS-CoV cell-cell fusion experiment as described above. Similar results were also obtained in the SARS virus and SARS-like pseudovirus inhibition experiments (Figures 4, 5, 6 and 7). The negative control 2019-HR1P polypeptide showed no activity.

The polypeptides or polypeptide derivatives of the present invention not only show a highly effective activity against 2019-nCoV, but also show their specific antiviral mechanism as exemplified by the 2019-HR2P polypeptide. In the Native PAGE experiment shown in Figure 8a, the 2019-HR2P polypeptide and EK1 polypeptide can be mixed with the 2019-HR1P derived from the 2019-nCoV HR1 region, and both produce a new band, which is a six-helix band. In addition, the detection of the secondary structure of polypeptides showed that when the 2019-HR2P polypeptide and EK1 polypeptide were mixed with the 2019-HR1P, a high degree of helical structure appeared. This result was consistent with the CD spectrum of the six-helix core structure, and its Tm value indicated its thermal stability (Figure 8b). It can be seen that the antiviral mechanism of the 2019-HR2P polypeptide is mainly through binding to the HR1 region of the 2019-nCoV virus to interfere with the viral 6HB formation, thereby inhibiting the fusion and entry processes of the virus.

In summary, the series of 2019-HR2P polypeptides have a good inhibitory activity against the 2019-nCoV, SARS viruses, and SARS-like viruses, providing a good preventive and therapeutic candidate drug for the currently circulating 2019-nCoV and the SARS-like viruses that may break out in the future. In addition, the clear antiviral mechanism of the 2019-HR2P polypeptide can ensure the safety of its application and the clarity of the optimization approach, which is convenient for further development in the future.

### Example 6: Inhibition of the SARS-CoV-2 live virus by EK1 and EK1-chol on the human intestinal cell line CaCO2

### (I) Experimental Methods:

1. This experiment was carried out in the biosafety level 3 laboratory of Fudan University, and the SARS-CoV-2 strain used was the nCoV-SH01 strain isolated in this laboratory.
2. CaCO2 cells were plated in a 96-well plate (10,000 cells per well) 12 hours before infection.
3. The polypeptide inhibitor was serially diluted using the serum-free DMEM with the volume of 50 µL, and 50 µL of virus diluent (750 pfu/ml) was then added, and mixed, followed by incubation at 37°C for 30 mins.
4. 100 µL of virus/drug mixture was added to the target cells that had been plated in a 96-well plate, and incubated with 5% CO2 at 37°C for 48 hours.
5. The supernatant of each well was collected and quantitatively detected for the amount of viral RNA in the supernatant to calculate the inhibition rate.

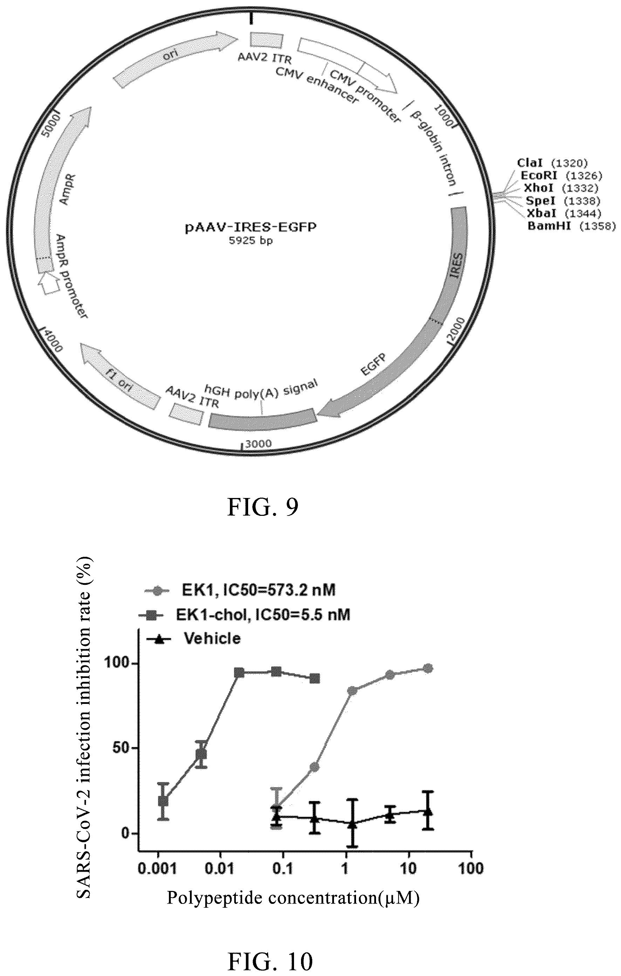

The results show that:
SARS-CoV-2 can effectively infect human intestinal tissues, so it is of great significance for us to use human intestinal cell lines to evaluate the polypeptides EK1 and EK1-chol. The results are shown in Figure 10 that both EK1 and EK1-chol polypeptides have highly effective inhibitory effects on SARS-CoV-2, and their half inhibitory concentrations are 573.2 nM and 5.5 nM, respectively.

### Example 7: In vivo protective effect of the polypeptide EK1 on transgenic mice infected with the SARS-CoV-2 live virus

### (I) Experimental Methods:

1. This experiment was carried out in the biosafety level 3 laboratory of Fudan University, and the SARS-CoV-2 strain used was the nCoV-SH01 strain isolated in this laboratory; the transgenic human-ACE2 mice were purchased from Shanghai Model Organisms Center, Inc.; Mouse strain: Tgtn (CAG-human ACE2-IRES-Luciferase).
2. Each mouse was challenged with 30,000 pfu SARS-CoV-2 (30 µL).
3. 30 minutes before the challenge; or 30 minutes after the challenge, the dose of 200 µg EK1/mouse was administered by intranasal route.
4. The mice were euthanized on the 4th day after infection, and the viral load of the lungs and intestinal tissues of mice in each group was detected.

### (II) The results show that:

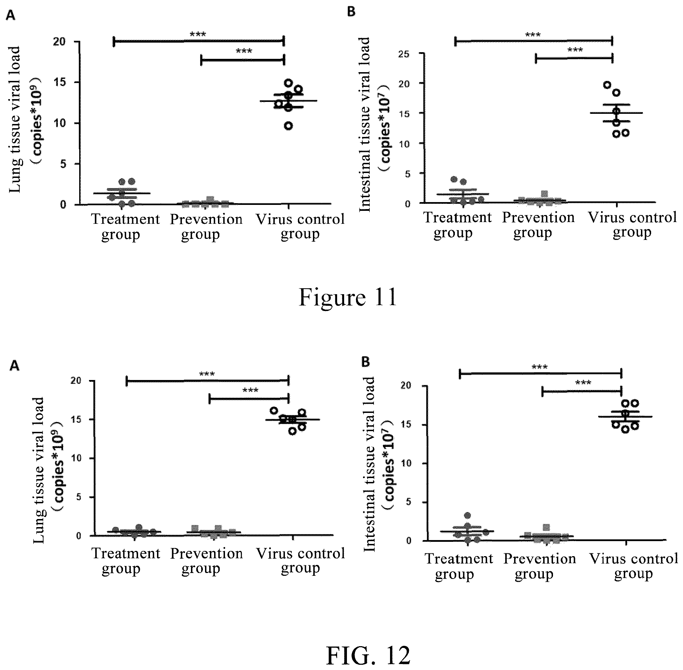

The therapeutic administration of the EK1 polypeptide through the respiratory tract by nose drops can significantly reduce the SARS-CoV-2 viral load in the lung (A) and intestine (B) (Figure 11), indicating that EK1 has a better protective effect in vivo.

### Example 8: In vivo protective effect of the polypeptide EK1-chol on transgenic mice infected with the SARS-CoV-2 live virus

### (I) Experimental Methods:

1. This experiment was carried out in the biosafety level 3 laboratory of Fudan University, and the SARS-CoV-2 strain used was the nCoV-SH01 strain isolated in this laboratory; the transgenic human-ACE2 mice were purchased from Shanghai Model Organisms Center, Inc.; Mouse strain: Tgtn (CAG-human ACE2-IRES-Luciferase).
2. Each mouse was challenged with 30,000 pfu SARS-CoV-2 (30 µL).
3. 30 minutes before the challenge; or 30 minutes after the challenge, the dose of 10 µg EK1C4/mouse was administered by intranasal route.
4. On the 4th day after infection, the mice were euthanized, and the viral load of the lungs and intestinal tissues of mice in each group was detected.

### (II) The results show that:

The therapeutic administration of the EK1-chol polypeptide through the respiratory tract by nose drops can significantly reduce the SARS-CoV-2 viral titer in the lung (A) and intestine (B) (Figure 12), indicating that EK1-chol has an effectively protective effect in vivo.

## Claims

1. A polypeptide set forth as SEQ ID NO. 2, a fusion protein or conjugate comprising the polypeptide, a nucleic acid encoding the polypeptide or the fusion protein, a vector comprising the nucleic acid, a host cell comprising the nucleic acid or a composition comprising the polypeptide for use in inhibiting 2019-nCoV or treating and/or preventing a disease caused by 2019-nCoV;
preferably the disease is coronavirus pneumonia;
preferably the dosage form of the medicament is tablet, capsule, dripping pill, aerosol, pill, powder, solution, suspension, emulsion, granule, liposome, transdermal agent, suppository or lyophilized powder injection;
preferably the medicament is administered by the followings: injection, cavity administration, respiratory administration, mucosal administration, or topical administration;
preferably the medicament is administered by the followings: subcutaneous injection, intravenous injection, intramuscular injection and intraperitoneal injection, intracisternal injection or infusion, rectal administration, vaginal administration, sublingual administration, or nasal cavity administration.

2. A polypeptide derivative of SEQ ID NO. 2, which is a derivative obtained by palmitoylation modification or cholesterol modification of the polypeptide, wherein the polypeptide is linked to palmitoylation modified lysine or cholesterol modified cysteine via a linker at the C-terminus, wherein the linker is PEGylated;
preferably, wherein the linker comprises (GSG)n or (GSGSG)n, wherein n is 1 or 2,
preferably, wherein the linker is -(GSG)n-DPEG4- or -(GSGSG)n-DPEG4-,
preferably, wherein the polypeptide derivative is SLDQINVTFLDLEYEMKKLEEAIKKLEESYIDLKEL-(GSG)n-DPEG4-K-palmitic acid or SLDQINVTFLDLEYEMKKLEEAIKKLEESYIDLKEL-(GSGSG)n-DPEG4-C-cholesterol, wherein n is 1 or 2.

3. A composition comprising the polypeptide derivative of claim 2; preferably, the composition is an external preparation, preferably an external liniment, such as an external gel or external infiltration preparation.

4. The composition of claim 3, further comprising an agent for inhibiting coronavirus or treating and/or preventing a disease caused by coronavirus, preferably selected from the group consisting of favipiravir, nelfinavir, arbidol, lopinavir, ritonavir, chloroquine phosphate, darunavir or remdesivir; preferably, the composition comprises a pharmaceutically acceptable carrier.

5. The polypeptide derivative of claim 2 or the composition of claim 3 or 4 for use in inhibiting coronavirus or treating and/or preventing a disease caused by coronavirus, such as coronavirus pneumonia,
preferably, wherein the coronavirus is 2019-nCoV, Rs3367-CoV or WIV1-CoV;
preferably, wherein the dosage form of the medicament is tablet, capsule, dripping pill, aerosol, pill, powder, solution, suspension, emulsion, granule, liposome, transdermal agent, suppository or lyophilized powder injection;
preferably, wherein the medicament is preferably administered by the followings: subcutaneous injection, intravenous injection, intramuscular injection and intraperitoneal injection, intracisternal injection or infusion, rectal administration, vaginal administration, sublingual administration, or nasal cavity administration.

6. The polypeptide derivative of claim 2, or the composition of claim 3 or 4 for use in inhibiting coronavirus in vitro,
preferably, wherein the coronavirus is 2019-nCoV, Rs3367-CoV or WIV1-CoV.

7. A polypeptide shown in SEQ ID NO. 2, a fusion protein or conjugate comprising the polypeptide, a nucleic acid encoding the polypeptide or the fusion protein, a vector comprising the nucleic acid, a host cell comprising the nucleic acid, a composition comprising the polypeptide for use in inhibiting 2019-nCoV in vitro.

## Patentansprüche

1. Polypeptid, das in SEQ ID Nr. 2 dargelegt ist, ein Fusionsprotein oder Konjugat, das das Polypeptid umfasst, eine Nukleinsäure, die das Polypeptid oder das Fusionsprotein kodiert, ein Vektor, der die Nukleinsäure umfasst, eine Wirtszelle, die die Nukleinsäure umfasst, oder eine Zusammensetzung, die das Polypeptid umfasst, zur Verwendung beim Hemmen von 2019-nCoV oder zum Behandeln und/oder Vorbeugen einer durch 2019-nCoV verursachten Krankheit;
wobei die Krankheit bevorzugt Coronavirus-Pneumonie ist;
wobei die Dosierungsform des Medikaments bevorzugt eine Tablette, eine Kapsel, eine Tropfpille, ein Aerosol, eine Pille, ein Pulver, eine Lösung, eine Suspension, eine Emulsion, ein Granulat, ein Liposom, ein transdermales Mittel, ein Zäpfchen oder eine Injektionsform aus gefriergetrocknetem Pulver ist;
wobei das Medikament bevorzugt auf folgende Weise verabreicht wird: Injektion, Verabreichung in die Körperhöhle, Verabreichung über die Atemwege, Verabreichung über die Schleimhäute oder topische Verabreichung;
wobei das Medikament bevorzugt auf folgende Weise verabreicht wird subkutane Injektion, intravenöse Injektion, intramuskuläre Injektion und intraperitoneale Injektion, intrazisternale Injektion oder Infusion, rektale Verabreichung, vaginale Verabreichung, sublinguale Verabreichung oder Verabreichung über die Nasenhöhle.

2. Polypeptidderivat von SEQ ID Nr. 2, das ein durch Palmitoylierungsmodifikation oder Cholesterinmodifikation des Polypeptids erhaltenes Derivat ist, wobei das Polypeptid über einen Linker am C-Terminus mit palmitoylierungsmodifiziertem Lysin oder cholesterinmodifiziertem Cystein verknüpft ist, wobei der Linker PEGyliert ist;
wobei der Linker bevorzugt (GSG)n oder (GSGSG)n umfasst, wobei n 1 oder 2 ist,
wobei der Linker bevorzugt -(GSG)n-DPEG4- oder -(GSGSG)n-DPEG4- ist,
wobei das Polypeptidderivat bevorzugt SLDQINVTFLDLEYEMKKLEEAIKKLEESYIDLKEL-(GSG)n-DPEG4-K-Palmitinsäure oder SLDQINVTFLDLEYEMKKLEEAIKKLEESYIDLKEL-(GSGSG)n-DPEG4-C-Cholesterin ist, wobei n 1 oder 2 ist.

3. Zusammensetzung, die das Polypeptidderivat nach Anspruch 2 umfasst;
wobei die Zusammensetzung bevorzugt ein äußerliches Präparat, bevorzugt ein äußerliches Einreibemittel, wie etwa ein äußerliches Gel oder ein äußerliches Infiltrationspräparat ist.

4. Zusammensetzung nach Anspruch 3, die weiter ein Mittel zum Hemmen von Coronavirus oder zum Behandeln und/oder Vorbeugen einer durch das Coronavirus verursachten Erkrankung umfasst, bevorzugt ausgewählt aus der Gruppe bestehend aus Favipiravir, Nelfinavir, Arbidol, lopinavir, Ritonavir, Chloroquinphosphat, Darunavir oder Remdesivir; wobei die Zusammensetzung bevorzugt einen pharmazeutisch verträglichen Träger umfasst.

5. Polypeptidderivat nach Anspruch 2 oder Zusammensetzung nach Anspruch 3 oder 4 zur Verwendung beim Hemmen von Coronavirus oder zum Behandeln und/oder Vorbeugen einer durch das Coronavirus verursachten Erkrankung, wie etwa einer Coronavirus-Pneumonie,
wobei das Coronavirus bevorzugt 2019-nCoV, Rs3367-CoV oder WIV1-CoV ist;
wobei die Dosierungsform des Medikaments bevorzugt eine Tablette, eine Kapsel, eine Tropfpille, ein Aerosol, eine Pille, ein Pulver, eine Lösung, eine Suspension, eine Emulsion, ein Granulat, ein Liposom, ein transdermales Mittel, ein Zäpfchen oder eine Injektionsform aus gefriergetrocknetem Pulver ist;
bevorzugt wobei das Medikament bevorzugt auf folgende Weise verabreicht wird: subkutane Injektion, intravenöse Injektion, intramuskuläre Injektion und intraperitoneale Injektion, intrazisternale Injektion oder Infusion, rektale Verabreichung, vaginale Verabreichung, sublinguale Verabreichung oder Verabreichung durch die Nasenhöhle.

6. Polypeptidderivat nach Anspruch 2 oder Zusammensetzung nach Anspruch 3 oder 4 zur Verwendung beim Hemmen von Coronavirus in vitro,
wobei das Coronavirus bevorzugt 2019-nCoV, Rs3367-CoV oder WIV1-CoV ist.

7. Polypeptid, das in SEQ ID Nr. 2 gezeigt ist, ein Fusionsprotein oder Konjugat, das das Polypeptid umfasst, eine Nukleinsäure, die das Polypeptid oder das Fusionsprotein kodiert, ein Vektor, der die Nukleinsäure umfasst, eine Wirtszelle, die die Nukleinsäure umfasst, eine Zusammensetzung, die das Polypeptid umfasst, zur Verwendung beim Hemmen von 2019-nCoV in vitro.

## Revendications

1. Polypeptide présenté par SEQ ID NO. 2, protéine de fusion ou conjugué comprenant le polypeptide, un acide nucléique codant pour le polypeptide ou la protéine de fusion, un vecteur comprenant l'acide nucléique, une cellule hôte comprenant l'acide nucléique ou composition comprenant le polypeptide pour utilisation pour inhiber le 2019-nCoV ou traiter et/ou prévenir une maladie causée par le 2019-nCoV ;
de préférence, la maladie est une pneumonie à coronavirus ;
de préférence, la forme posologique du médicament est un comprimé, une capsule, une pilule goutte à goutte, un aérosol, une pilule, une poudre, une solution, une suspension, une émulsion, un granulé, un liposome, un agent transdermique, un suppositoire ou une injection de poudre lyophilisée ;
de préférence, le médicament est administré par les voies suivantes : injection, administration par cavité, administration respiratoire, administration par muqueuse ou administration topique ;
de préférence, le médicament est administré par les voies suivantes : injection sous-cutanée, injection intraveineuse, injection intramusculaire et injection intrapéritonéale, injection ou perfusion intracisternale, administration rectale, administration vaginale, administration sublinguale ou administration par la cavité nasale.

2. Dérivé polypeptidique de SEQ ID NO. 2, qui est un dérivé obtenu par modification par palmitoylation ou modification par cholestérol du polypeptide, dans lequel le polypeptide est lié à de la lysine modifiée par palmitoylation ou de la cystéine modifiée par cholestérol via un lieur à l'extrémité C-terminale, dans lequel le lieur est PEGylé ;
de préférence, dans lequel le lieur comprend (GSG)n ou (GSGSG)n, dans lequel n est 1 ou 2,
de préférence, dans lequel le lieur est -(GSG)n-DPEG4- ou -(GSGSG)n-DPEG4-,
de préférence, dans lequel le dérivé polypeptidique est l'acide SLDQINVTFLDLEYEMKKLEEAIKKLEESYIDLKEL-(GSG)n-DPEG4-K-palmitique ou le SLDQINVTFLDLEYEMKKLEEAIKKLEESYIDLKEL-(GSGSG)n-DPEG4-C-cholestérol, dans lequel n est 1 ou 2.

3. Composition comprenant le dérivé polypeptidique selon la revendication 2 ;
de préférence la composition est une préparation externe, de préférence un liniment externe, tel qu'un gel externe ou une préparation d'infiltration externe.

4. Composition selon la revendication 3, comprenant en outre un agent pour inhiber un coronavirus ou traiter et/ou prévenir une maladie causée par un coronavirus, de préférence choisi dans le groupe consistant en le favipiravir, le nelfinavir, l'arbidol, le lopinavir, le ritonavir, le phosphate de chloroquine, le darunavir ou le remdésivir ; de préférence, la composition comprend un support pharmaceutiquement acceptable.

5. Dérivé polypeptidique selon la revendication 2 ou composition selon la revendication 3 ou 4 pour utilisation pour inhiber un coronavirus ou traiter et/ou prévenir une maladie causée par un coronavirus, comme la pneumonie à coronavirus,
de préférence dans lequel le coronavirus est 2019-nCoV, Rs3367-CoV ou WIV1-CoV ;
de préférence dans lequel la forme posologique du médicament est un comprimé, une capsule, une pilule goutte à goutte, un aérosol, une pilule, une poudre, une solution, une suspension, une émulsion, un granulé, un liposome, un agent transdermique, un suppositoire ou une injection de poudre lyophilisée ;
de préférence dans lequel le médicament est administré de préférence par les voies suivantes : injection sous-cutanée, injection intraveineuse, injection intramusculaire et injection intrapéritonéale, injection ou perfusion intracisternale, administration rectale, administration vaginale, administration sublinguale ou administration par la cavité nasale.

6. Dérivé polypeptidique selon la revendication 2, ou composition selon la revendication 3 ou 4, pour utilisation pour inhiber un coronavirus in vitro,
de préférence dans lequel le coronavirus est 2019-nCoV, Rs3367-CoV ou WIV1-CoV.

7. Polypeptide représenté dans SEQ ID NO. 2, protéine de fusion ou conjugué comprenant le polypeptide, acide nucléique codant pour le polypeptide ou la protéine de fusion, vecteur comprenant l'acide nucléique, cellule hôte comprenant l'acide nucléique, une composition comprenant le polypeptide pour utilisation pour inhiber le 2019-nCoV in vitro.
